# EUROPEAN PATENT SPECIFICATION

(11) **EP 3 193 963 B1**
(45) Date of publication and mention of the grant of the patent: **31.07.2019**
(21) Application number: 15842917.5
(22) Date of filing: 18.09.2015
(51) Int. Cl.: A61M 1/14, B01D 61/28, B01D 61/32, A61M 1/16

(54) **DIALYSIS MACHINE HAVING A CONDUCTIVITY SENSOR FOR DETERMINING FLUID PROPERTIES**
DIALYSEMASCHINE MIT EINEM LEITFÄHIGKEITSSENSOR ZUR BESTIMMUNG VON FLÜSSIGKEITSEIGENSCHAFTEN
MACHINE DE DIALYSE AYANT UN CAPTEUR DE CONDUCTIVITÉ POUR DÉTERMINER LES PROPRIÉTÉS D'UN FLUIDE

(30) Priority: 18.09.2014 US 201462052410 P
(43) Date of publication of application: 26.07.2017
(73) Proprietor: Outset Medical, Inc., San Jose, CA 95112 (US)
(72) Inventor: MILLER, Steven Michael, San Jose, CA 95112 (US); HSU, George Chao-Chih, San Jose, CA 95112 (US); BRIDGES, Paul Thomas, San Jose, CA 95112 (US); STIENMIER, John David, San Jose, CA 95112 (US)
(74) Representative: J A Kemp
(86) International application number: PCT/US2015/051011
(87) International publication number: WO 2016/044760

(56) References cited:
- US-A- 3 710 237
- US-A- 3 710 237
- US-A- 6 058 934
- US-A- 6 058 934
- US-A1- 2009 012 452
- US-A1- 2010 321 046
- US-A1- 2014 018 727

## Description

### FIELD

This disclosure is generally related to dialysis machines and therapy. This disclosure is more specifically related to conductivity sensors in dialysis machines.

### BACKGROUND

Some patients receive dialysis treatment at a dialysis center, which can place a demanding, restrictive and tiring schedule on a patient. Patients who receive in-center dialysis may have to travel to the dialysis center at least three times a week and sit in a chair for 3 to 4 hours each time while toxins and excess fluids are filtered from their blood. After the treatment, the patient may be required to wait for the needle site to stop bleeding and blood pressure to return to normal, which can require even more time taken away from other activities in their daily lives. In contrast to dialysis center treatments, home dialysis can provide patients with scheduling flexibility as it permits patients to choose treatment times.

US 6 058 934 A discloses a conductivity sensor for measuring hematocrit and a sensor housing for a blood analysis instrument using the conductivity sensor. The conductivity sensor includes a seven-electrode conductivity measurement cell in which three symmetric pairs of electrodes are arranged on opposite sides of a central electrode. The central electrode is connected to an AC source and the outermost pair of electrodes, which provide a return path for the current, are maintained at a ground or reference potential. The two inner pairs of electrodes measure the voltage drop along the current flow path.

US 3 710 237 A discloses a probe for a conductivity testing device.

### SUMMARY OF THE DISCLOSURE

According to the present invention there is provided the conductivity sensor of claim 1. Additional aspects of the invention are set out in the dependent claims.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 illustrates an example dialysis system that can provide dialysis treatment to a user in a non-clinical setting, such as in the user's home.
FIG. 2 shows an embodiment of a conductivity sensor having a two-pole configuration that can be included in the dialysis system of FIG. 1.
FIG. 3 shows an example graph of a prospective error-to-conductivity relationship of a conductivity sensor that has a single cell constant, such as the conductivity sensor shown in FIG. 2.
FIG. 4 shows another embodiment of a conductivity sensor that can be included in the dialysis system of FIG. 1 and includes a seven-electrode configuration.
FIG. 5 shows an example graph that compares conductivity measurements that can be achieved by a conductivity sensor having a single cell constant with conductivity measurements that can be achieved by a conductivity sensor having different cell constants, such as the conductivity sensor shown in FIG. 4.
FIG. 6 illustrates another embodiment of a conductivity sensor having a seven-electrode configuration and amplifiers for measuring voltage across sense electrodes.

### DETAILED DESCRIPTION

This disclosure describes systems, devices and methods related to embodiments of a dialysis system, including a home dialysis system, having a conductivity sensor or sensing system that measures conductivity in fluid associated with the dialysis system. For example, the conductivity sensor of the dialysis system can measure the conductivity of dialysate in order to determine its composition and verify proportioning of the dialysate. The conductivity sensor can also measure the purity of water and verify its proportioning in the dialysis system. In addition, the conductivity sensor can be configured to detect conductivity of the fluid within an acceptable range, as will be described in greater detail below.

The conductivity sensor can include drive electrodes (i.e., drives current) and sense electrodes (i.e., measures voltage). The drive electrodes can be powered by an alternating voltage, and current that flows from the drive electrodes through the fluid can be measured by the sense electrodes to determine the conductivity of the fluid. The conductivity of the fluid can be affected by one or more characteristics of the fluid, such as chemical composition and temperature.

For example, the conductivity sensor can drive a current through the fluid and measure a drop in voltage across the fluid. Based on the current, voltage, distance between sense electrodes and effective area between the sense electrodes, the conductivity sensor can calculate the conductivity across the fluid.

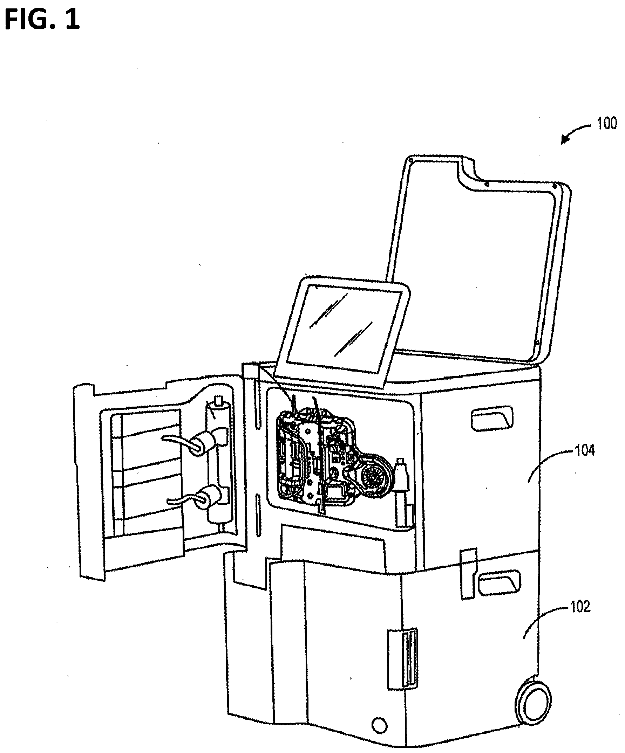

FIG. 1 illustrates an example dialysis system 100 that can provide dialysis treatment to a user in a non-clinical setting, such as in the user's home. The dialysis system 100 can include a water purification system (WPS) module 102 and a dialysis delivery system (DDS) module 104 that can be reversibly coupled together, such as for improved transport and storage. The WPS module 102 can assist in purifying a water source for use with dialysis treatment. For example, the WPS module 102 can be connected to a residential water source (e.g., tap water) and prepare pasteurized water in at least near real-time. The pasteurized water can then be used for dialysis (e.g., in the DDS module 104) in a non-clinical setting without the need to heat and cool large, batched quantities of water. One or more conductivity sensors, including any of the conductivity sensors described herein can be included in the dialysis system 100 for measuring conductivity of a fluid associated with the dialysis system 100, such as dialysate or water.

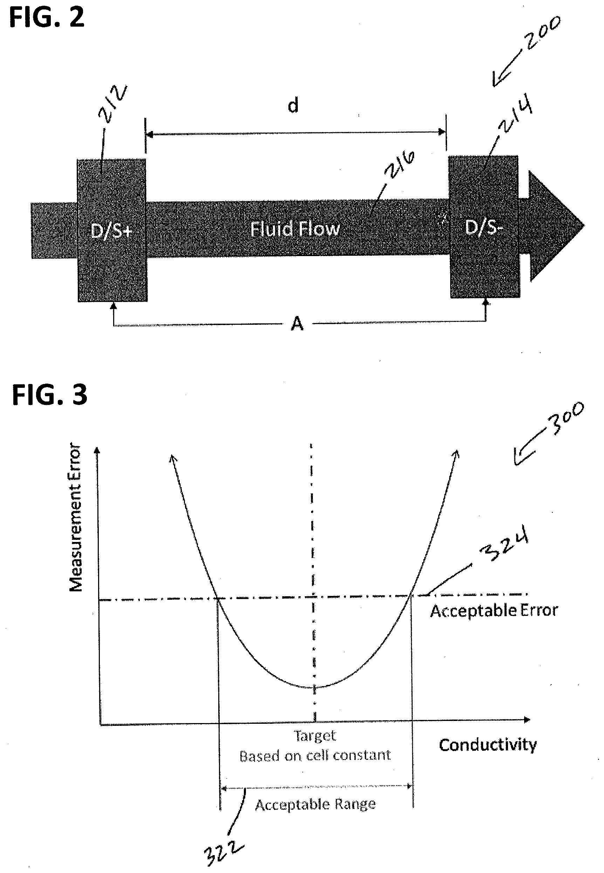

FIG. 2 illustrates a conductivity sensor 200 having a two-pole configuration for use in a dialysis system. The conductivity sensor 200 can include a positive drive/sense electrode 212 and a negative drive/sense electrode 214. A current can be driven across the fluid 216 (e.g., dialysate, water) between the drive/sense electrodes 212, 214 and a voltage drop can be measured between the drive/sense electrodes 212, 214. The conductivity sensor 200 can calculate the conductivity of the fluid 216 based on the current, voltage, distance (d) between the drive/sense electrodes 212, 214 and an effective area (A) between the drive/sense electrodes 212, 214. For example, the distance and effective area between the drive/sense electrodes 212, 214 can be used to calculate a cell constant, which can be used to calculate the conductivity. In addition, the variables of the cell constant (i.e., d and A) can be tuned so as to allow the conductivity sensor 200 to measure conductivity of the fluid 216 within an acceptable range (i.e., relatively low measurement error).

FIG. 3 illustrates an example graph 300 of a prospective error-to-conductivity relationship of a conductivity sensor, such as conductivity sensor 200, which has a single cell constant. As shown in the graph 300, an acceptable range 322 can define conductivity measurements that can be achieved by the conductivity sensor that are below an acceptable error threshold 324. The acceptable range 322 can be based on the cell constant of the conductivity sensor. As such, the cell constant can be tuned (e.g., the d and/or A adjusted) in order to achieve a desired range of conductivity measurements that are within the acceptable range 322.

A number of variables in the dialysis system can interfere with conductivity measurements such that a more advanced conductivity sensor is needed. For example, electrical noise from other parts of the dialysis system and leakage currents can interfere with measuring conductivity of fluids within the dialysis system.

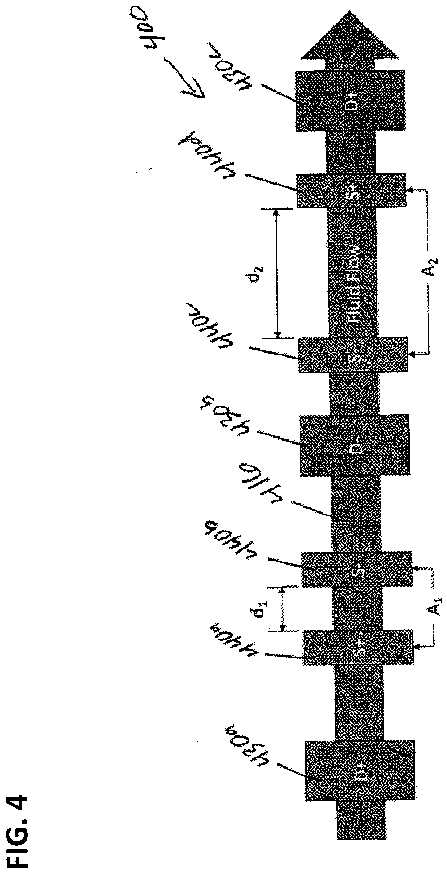

FIG. 4 illustrates an embodiment of a conductivity sensor 400 having a seven-electrode configuration that separates pairs of drive electrodes and sense electrodes. This configuration can provide several advantages over other conductivity sensors, including conductivity sensor 200 of FIG. 2, such as by shielding the conductivity sensor 400 from electrical noise and variable ground paths.

As shown in FIG. 4, the conductivity sensor 400 can include a first drive electrode 430a, a second drive electrode 430b, and a third drive electrode 430c configured to drive current through a fluid 416 of the dialysis system. In addition, first and second sense electrodes 440a and 440b can be positioned between the first and second drive electrodes 430a and 430b. Furthermore, third and fourth sense electrodes 440c and 440d can be positioned between the second and third drive electrodes 430b and 430c. In this configuration, each pair of sense electrodes (i.e., 440a and 440b, 440c and 440d) can have a cell constant, which is defined as the ratio of the distance between the electrodes to the electrode area. Furthermore, the first pair of sense electrodes 440a and 440b can have a different cell constant than the second pair of sense electrodes 440c and 440d.

As described above, a cell constant is the ratio of the distance between electrodes to the electrode area. The conductivity sensor 400 can have distinct cell constants between the pairs of sense electrodes as follows: For example, a first distance (dl) between the first and second sense electrodes 440a and 440b can be different than a second distance (d2) between the third and fourth sense electrodes 440c and 440d. Alternatively or in addition, a first area (A1) of the first and second sense electrodes 440a and 440b can be different than a second area (A2) of the third and fourth sense electrodes 440c and 440d. This can result in the cell constant created by the first and second sense electrodes 440a and 440b to be different than the cell constant created by the third and fourth sense electrodes 440c and 440d. The conductivity sensor 400 includes two pairs of electrodes with different cell constants, whereby the combination of these two different cell constants allows the conductivity sensor 400 to measure a wider range of conductivity measurements that are below an acceptable error threshold, as will be explained in greater detail below.

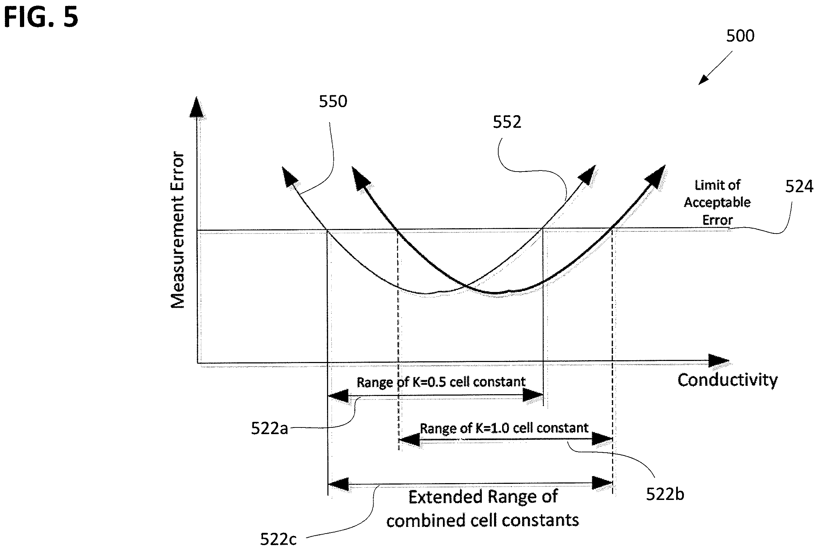

FIG. 5 shows an example graph 500 that shows conductivity measurements that can be achieved by a conductivity sensor, such as conductivity sensor 400, having different cell constants (i.e., first conductivity line 550 and second conductivity line 552). As can be seen in graph 500, the first conductivity line 550 has a first conductivity range 522a, and the second conductivity line 552 has a second conductivity range 522b. The first and second conductivity ranges are combined to form an extended conductivity range 522c that falls below the limit of acceptable error for the conductivity sensor 400. When the two conductivity lines are combined, the conductivity sensor has a larger acceptable range 522c of conductivity measurements that can be collected below the acceptable error threshold 524 compared to the smaller acceptable ranges 522a and 522b the individual cell constants. As such, the conductivity sensor 400 having different cell constants can measure a greater range of conductivity measurements that have acceptable errors.

The embodiment illustrated in FIG. 5 shows a conductivity sensor having a first cell constant of K = 0.5 and a second cell constant of K = 1.0. As the conductivity decreases (increasing resistance) a given current will produce a larger voltage signal. At some point the signal becomes so large that the amplifier circuit has reached its upper voltage limit. But a conductivity censor having a first cell constant that is half the size of the second cell constant cuts the voltage signal in half, effectively doubling the lower range of the sensor. For example, if the practical lower limit of a sensor with K = 1.0 is 100uS, then a sensor with a K of 0.5 has a practical lower limit of 50uS.

Cell constants in conductivity sensor 400 can be tuned by either changing the distance or effective area between the pairs of sense electrodes in order to adjust the acceptable conductivity measurement range of the conductivity sensor 400. This can allow the conductivity sensor 400 to collect a broad range of conductivity measurements that are appropriate for the type of fluid being measured, along with the measurements being below the acceptable error threshold 524.

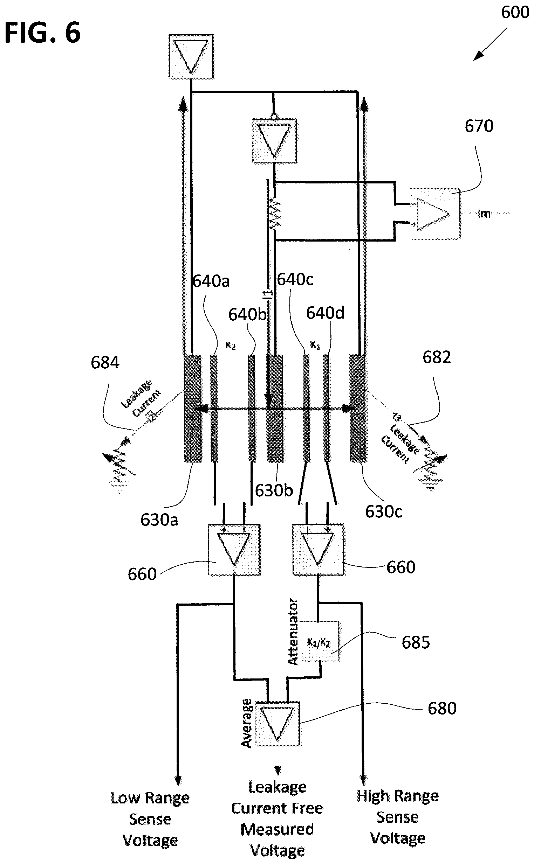

FIG. 6 illustrates another embodiment of a conductivity sensor 600 having a seven-electrode configuration. For example, the conductivity sensor 600 includes first and second sense electrodes 640a and 640b positioned between first and second drive electrodes 630a and 630b. In addition, the conductivity sensor 600 includes third and fourth sense electrodes 640c and 640d positioned between the second drive electrode 630b and a third drive electrode 630c. The cell constant of the first and second sense electrodes 640a and 640b can be different from the cell constant of the third and fourth sense electrodes 640c and 640d. First amplifiers 660 can assist in independently measuring the voltage sensed by the pairs of sense electrodes (i.e., 640a and 640b, 640c and 640d). A second amplifier 680 can process and combine the independently measured voltage readings, such as from the first amplifiers 660. The resulting signal from the second amplifier, or averaging amplifier 680, can be further processed to produce a single conductivity reading. The measurement error of the conductivity sensor 600 can be the combination of measurement errors associated with each pair of sense electrodes. In addition, the seven-electrode configuration limits alteration of the conductivity reading obtained by the conductivity sensor 600 by external effect, such as from noise current 682 and leakage current 684. With different cell constants within the conductivity sensor, an additional attenuation element 685 can be used to achieve effectively scaled current subtraction with the averaging amplifier 680.

In some embodiments, additional electronics (e.g., sensors) can be included in the conductivity sensor 600 in order to ensure proper functioning. For example, conductivity sensor 600 can include a sensor 670 for measuring current along a section of the electrical pathway in order to ensure that the current is staying within a preferred range. The preferred range can be a single preset value or a dynamically tuned value that assists the conductivity sensor 600 with achieving precise conductivity calculations (i.e., minimize individual and combined measurement errors for voltage and current).

In addition, by independently measuring voltage across the pairs of sense electrodes, the conductivity sensor 600 can allow the sense electrodes to be placed in a variety of positions relative to each other between corresponding drive electrodes. Additionally, increasing the difference in cell constants in the conductivity sensor 600 can increase the acceptable range. The noise level can be decreased by decreasing the difference in cell constants.

A target conductivity range of water and dialysate, for example, can be from approximately 0 uS/cm to 1,000 uS/cm and approximately 8,000 uS/cm to 20,000 uS/cm, respectively. In addition, target cell constants for each pair of sense electrodes can be approximately 0.1 to 1.0 and approximately 1.0 to 5.0.

While this specification contains many specifics, these should not be construed as limitations on the scope of the claims, but rather as descriptions of features specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or a variation of a sub-combination. Similarly, while operations are depicted in the drawings in a particular order, this should not be understood as requiring that such operations be performed in the particular order shown or in sequential order, or that all illustrated operations be performed, to achieve desirable results. Only a few examples and implementations are disclosed. Variations, modifications and enhancements to the described examples and implementations and other implementations may be made based on what is disclosed.

As for additional details pertinent to the present invention, materials and manufacturing techniques may be employed as within the level of those with skill in the relevant art. The same may hold true with respect to method-based aspects of the invention in terms of additional acts commonly or logically employed. Also, it is contemplated that any optional feature of the inventive variations described may be set forth and claimed independently, or in combination with any one or more of the features described herein. Likewise, reference to a singular item, includes the possibility that there are plural of the same items present. More specifically, as used herein and in the appended claims, the singular forms "a," "and," "said," and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Unless defined otherwise herein, all technical and scientific terms used herein have the same meaning as commonly understood by one of ordinary skill in the art to which this invention belongs. The breadth of the present invention is not to be limited by the subject specification, but rather only by the plain meaning of the claim terms employed.

## Claims

1. A conductivity sensor, comprising:
a first pair of sense electrodes having a first cell constant;
a second pair of sense electrodes having a second cell constant different than the first cell constant;
wherein the first and second pairs of sense electrodes are each configured to measure a conductivity of a fluid, and wherein the first and second pairs of sense electrodes combine to increase a range of conductivity measurements that fall below an acceptable error threshold;
wherein the conductivity sensor is disposed within a dialysis system;
wherein the first cell constant ranges from 0.1 to 1.0 and the second cell constant ranges from 1.0 to 5.0;
wherein the first pair of sense electrodes comprises a first sense electrode and a second sense electrode, and the second pair of sense electrodes comprises a third sense electrode and a fourth sense electrode; and
wherein the first and second sense electrodes are positioned between a first drive electrode and a second drive electrode, and the third and fourth sense electrodes are positioned between the second drive electrode and a third drive electrode.

2. The conductivity sensor of claim 1, further comprising a first amplifier configured to measure voltages produced by the first and second pairs of sense electrodes.

3. The conductivity sensor of claim 2, further comprising a second amplifier configured to combine the measured voltages from the first amplifier.

4. The conductivity sensor of claim 3, further comprising an attenuation element configured to achieve scaled current subtraction with the second amplifier.

## Patentansprüche

1. Leitfähigkeitssensor, umfassend:
ein erstes Paar Sensorelektroden mit einer ersten Zellenkonstanten;
ein zweites Paar Sensorelektroden mit einer zweiten Zellenkonstanten, die sich von der ersten Zellenkonstanten unterscheidet;
wobei das erste und zweite Paar Sensorelektroden jeweils konfiguriert sind, eine Leitfähigkeit eines Fluids zu messen, und wobei sich das erste und zweite Paar Sensorelektroden verbinden, um einen Bereich von Leitfähigkeitsmessungen, die unter einen annehmbaren Fehlerschwellwert fallen, zu erhöhen;
wobei der Leitfähigkeitssensor in einem Dialysesystem angeordnet ist;
wobei die erste Zellenkonstante im Bereich von 0,1 bis 1,0 liegt und die zweite Zellenkonstante in einem Bereich von 1,0 bis 5,0 liegt;
wobei das erste Paar Sensorelektroden eine erste Sensorelektrode und eine zweite Sensorelektrode umfasst und das zweite Paar Sensorelektroden eine dritte Sensorelektrode und eine vierte Sensorelektrode umfasst; und
wobei die erste und zweite Sensorelektrode zwischen einer ersten Ansteuerelektrode und einer zweiten Ansteuerelektrode positioniert sind und die dritte und vierte Sensorelektrode zwischen der zweiten Ansteuerelektrode und einer dritten Ansteuerelektrode positioniert sind.

2. Leitfähigkeitssensor nach Anspruch 1, ferner umfassend einen ersten Verstärker, der konfiguriert ist, Spannungen, die durch das erste und zweite Paar Sensorelektroden erzeugt sind, zu messen.

3. Leitfähigkeitssensor nach Anspruch 2, ferner umfassend einen zweiten Verstärker, der konfiguriert ist, die gemessenen Spannungen von dem ersten Verstärker zu kombinieren.

4. Leitfähigkeitssensor nach Anspruch 3, ferner umfassend ein Dämpfungselement, das konfiguriert ist, skalierte Stromsubtraktion mit dem zweiten Verstärker zu erreichen.

## Revendications

1. Capteur de conductivité, comprenant :
une première paire d'électrodes de détection présentant une première constante de cellule ;
une deuxième paire d'électrodes de détection présentant une deuxième constante de cellule différente de la première constante de cellule ;
dans lequel les première et deuxième paires d'électrodes de détection sont chacune conçues pour mesurer la conductivité d'un fluide, et dans lequel les première et deuxième paires d'électrodes de détection se combinent pour augmenter une plage de mesures de conductivité qui se situent au-dessous d'un seuil d'erreur acceptable ;
dans lequel le capteur de conductivité est disposé dans un système de dialyse ;
dans lequel la première constante de cellule va de 0,1 à 1,0 et la deuxième constante de cellule va de 1,0 à 5,0 ;
dans lequel la première paire d'électrodes de détection comprend une première électrode de détection et une deuxième électrode de détection, et la deuxième paire d'électrodes de détection comprend une troisième électrode de détection et une quatrième électrode de détection ; et
dans lequel les première et deuxième électrodes de détection sont positionnées entre une première électrode d'excitation et une deuxième électrode d'excitation, et les troisième et quatrième électrodes de détection sont positionnées entre la deuxième électrode d'excitation et une troisième électrode d'excitation.

2. Capteur de conductivité selon la revendication 1, comprenant en outre un premier amplificateur conçu pour mesurer des tensions produites par les première et deuxième paires d'électrodes de détection.

3. Capteur de conductivité selon la revendication 2, comprenant en outre un deuxième amplificateur conçu pour combiner les tensions mesurées à partir du premier amplificateur.

4. Capteur de conductivité selon la revendication 3, comprenant en outre un élément d'atténuation conçu pour obtenir une soustraction de courant pondéré avec le deuxième amplificateur.
